(19)

Europäisches Patentamt

European Patent Office

Office européen des brevets

(11)  **EP 3 600 239 B1**

(12)  **EUROPEAN PATENT SPECIFICATION**

(45) Date of publication and mention
of the grant of the patent:
**30.10.2024  Bulletin 2024/44**

(21) Application number: **18714746.7**

(22) Date of filing: **26.03.2018**

(51) International Patent Classification (IPC):
***A61K 8/67*** *(2006.01)*        ***A61Q 17/04*** *(2006.01)*

(52) Cooperative Patent Classification (CPC):
**A61Q 17/04; A61K 8/673; A61K 8/675**

(86) International application number:
**PCT/EP2018/057577**

(87) International publication number:
**WO 2018/177969 (04.10.2018 Gazette 2018/40)**

(54) **METHOD OF PROTECTING HUMAN SKIN AGAINST DAMAGE UPON EXPOSURE WITH BLUE LIGHT**

VERFAHREN ZUM SCHUTZ DER MENSCHLICHEN HAUT GEGEN BESCHÄDIGUNG BEI BELICHTUNG MIT BLAULICHT

PROCÉDÉ DE PROTECTION DE LA PEAU HUMAINE CONTRE LES DOMMAGES LORS DE L'EXPOSITION À LA LUMIÈRE BLEUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2017  EP 17164106**

(43) Date of publication of application:
**05.02.2020  Bulletin 2020/06**

(73) Proprietor: **DSM IP Assets B.V.**
**6221 BE Maastricht (NL)**

(72) Inventors:
• **MENDROK-EDINGER, Christine**
**4303 Kaiseraugst (CH)**
• **RUDOLPH, Thomas**
**4303 Kaiseraugst (CH)**
• **STRAUSS, Karolina**
**4303 Kaiseraugst (CH)**

(74) Representative: **dsm-firmenich IP**
**Wurmisweg 576**
**4303 Kaiseraugst (CH)**

(56) References cited:
**WO-A1-97/30690      US-A1- 2005 100 517**

• **COATS JAHNNA G. ET AL: "Blue Light Protection, Part I-Effects of blue light on the skin", JOURNAL OF COSMETIC DERMATOLOGY, vol. 20, no. 3, 28 November 2020 (2020-11-28), GB, pages 714 - 717, XP055787721, ISSN: 1473-2130, Retrieved from the Internet <URL:https://onlinelibrary.wiley.com/doi/full-xml/10.1111/jocd.13837> DOI: 10.1111/jocd.13837**
• **ARJMANDI N ET AL: "Can Light Emitted from Smartphone Screens and Taking Selfies Cause Premature Aging and Wrinkles?", JOURNAL OF BIOMEDICAL PHYSICS AND ENGINEERING, 1 December 2018 (2018-12-01), Iran, pages 447 - 452, XP055781572, Retrieved from the Internet <URL:https://www.ncbi.nlm.nih.gov/pmc/articles/PMC6280109/pdf/JBPE-8-447.pdf> [retrieved on 20210303], DOI: 10.31661/jbpe.v0i0.599**
• **DATABASE GNPD [online] MINTEL; June 2014 (2014-06-01), "UV-Defence SPF 50+", XP002772461, Database accession no. 2573883**
• **DATABASE GNPD [online] MINTEL; May 2017 (2017-05-01), "Solar Defence Tinted Broad Spectrum SPF 30", XP002772462, Database accession no. 4785637**
• **DATABASE GNPD [online] MINTEL; May 2008 (2008-05-01), "Protective SPF 20", XP002772463, Database accession no. 919981**

- **ZUSSMAN J ET AL: "Vitamins and photoaging: Do scientific data support their use?", JOURNAL OF THE AMERICAN ACADEMY OF DERMATOLOGY, MOSBY, INC, US, vol. 63, no. 3, 1 September 2010 (2010-09-01), pages 507 - 525, XP027202646, ISSN: 0190-9622, [retrieved on 20100301]**
- **HAKOZAKI T ET AL: "The effect of niacinamide on reducing cutaneous pigmentation and suppression of melanosome transfer", BRITISH JOURNAL OF DERMATOLOGY, OXFORD : WILEY-BLACKWELL, UK, vol. 147, no. 1, 1 July 2002 (2002-07-01), pages 20 - 31, XP002333293, ISSN: 0007-0963, DOI: 10.1046/J.1365-2133.2002.04834.X**

**Description**

The present invention relates to the non-therapeutic use of a composition as defined in the appended set of claims for the protection of human skin against blue light damages.

The deleterious effects of skin exposure to ultraviolet (UV) radiation (both UVA and UVB) are well known. The UV radiation is categorized into three regions, UVC, UVB and UVA. Most of the UVC is filtered by ozone layer and high energy low wavelength UVB get absorbed in the upper layers of skin, i.e. epidermal region, while UVA penetrates little deeper into dermal regions of the skin. So far, most of the studies on skin exposure to light were concentrated on the role of UV irradiation due to its high energy, photo reactivity and its associated damage to the skin while the role of visible light has been less extensively investigated. In recent times, however, the adverse effects of visible light on skin has become more and more apparent.

Visible light is the region of light with 400-700 nm in the electromagnetic spectrum. Blue light is the portion of the electromagnetic spectrum in the visible region with wavelengths ranging from 400-500 nm. The wavelengths of blue light are close to UVA spectrum (315-400 nm) and the blue region of the visible spectrum is particularly important because it has a relatively high energy and at the same time longer wavelengths that can thus penetrate tissue deeper than UV light. It has recently been shown that blue light induces Reactive Oxygen Species (ROS) and Matrix-Degrading Enzymes in the skin which may lead inter alia to mitochondrial DNA damage and collagen degradation. Additionally, ROS generated by UV and blue light can cause further damage to proteins by oxidation of lysine, arginine, proline, and threonine residues. In addition, carbonyl groups may be introduced into proteins by reactions with aldehydes (4-hydroxy-2-nonenal, malondialdehyde) produced during lipid peroxidation or with reactive carbonyl derivatives generated as a consequence of the reaction of reducing sugars or their oxidation products with lysine residues of proteins. Both ROS and carbonyl groups on proteins (carbonylated proteins) can easily be measured in cells and tissue by a person skilled in the art and serve as markers for cell damage.

One defense system of the skin against reactive oxygen species (ROS) are radical scavenging substances like beta-carotene or lycopene which are also present in the skin. These antioxidants provide protection against ROS but they are themselves sensitive against electromagnetic irradiation and may thus serve as marker for skin damage.

Commercial products comprising vitamin B3 and vitamin B6 for the protection of human skin against photo-induced ageing or UV/IR are known.

WO97/30690 discloses a sunscreen composition to protect skin from the damages caused by exposure to sun, which composition comprising niacin and vitamin B6.

US2005/100517 discloses a skin lightening composition based on vitamin B3 or its derivatives and vitamin B6 or its derivatives.

Zussmann discloses the use of vitamins and photoageing (Journal of the American Academy of Dermatology, 2010, vol. 63, no. 3, pages 507-525).

The effect of niacinamide on reducing cutaneous pigmentation and suppression of melanosome transfer is disclosed by Hakozaki et al. (British Journal of Dermatology, 2002, vol 147, no. 1, pages 20-31.

Based on the more and more apparent adverse effects of electromagnetic radiation such as in particular visible light, there is an ongoing need for agents, which can protect the skin of the adverse effects of electromagnetic radiation such as in particular visible light.

Surprisingly, it has been found that the combination of niacinamide and pyridoxine hydrochloride synergistically protects the skin against the adverse effects of electromagnetic radiation such as in particular visible light.

Thus, in one aspect, the present invention relates to the non-therapeutic use of a cosmetic composition containing an effective amount of Vitamin B3 and an effective amount of Vitamin B6 for protecting human skin against damage upon exposure to blue light.

In an advantageous embodiment, the present invention relates to the non-therapeutic use of a cosmetic composition containing an effective amount of niacinamide and pyridoxine hydrochloride for reducing the generation of reactive oxygen species and/or the formation of carbonylated proteins in humans when exposed to blue light.

The term 'electromagnetic radiation' preferably refers to electromagnetic radiation between 290nm and 3.0 $\mu$m, preferably between 290nm to 800nm, most preferably between 400 nm to 500 nm. Such radiation can be emitted from natural sources as well as from artificial devices such as electronic displays. In all embodiments of the present invention, the term 'electromagnetic radiation' refers to blue light.

The term 'damage upon exposure to electromagnetic radiation as well as 'adverse effects of electromagnetic radiation, such as in particular visible or even blue light' as used herein encompasses in particular the generation of reactive species such as the formation of reactive oxygen species, reactive nitrogen species (RNS) and reactive carbonyl species (RCS) as well as the celluar damage thereof. Reactive species include radical and non-radical compounds such as nitroxyl-, carbonyl-, hydroxy- or oxyl-radicals, peroxylradicals, peroxides, superoxideradicalanion, hydrogenperoxide, singlet oxygen, lipoperoxides such as squaleneperoxides, ozone, nitrogene oxydes and NO-radical.

The term Vitamin B$_3$ used herein preferable refers Niacinamide [CAS-Nr. 98-92-0], which is one of the water-soluble B-complex vitamins, niacin [CAS-Nr. 59-67-6], which is also known as nicotinic acid and nicotinamid riboside. Niacinamide is also referred to as nicotinamide or pyridine-3-carboxamide and is the amide of niacin (vitamin $B_3$) and is e.g. available as Niacinamide PC or Niacinamide from DSM Nutritional Products AG, (4303 Kaiseraugst, Switzerland). Particularly preferred in all embodiments of the present invention is the use of niacinamide.

The term Vitamin $B_6$ refers in particular to pyridoxine hydrochloride [58-56-0], pyridoxal [CAS-Nr. 66-72-8] and pyridoxamin [CAS-Nr. 85-87-0]. In all embodiments, particularly preferred is the use of pyridoxine hydrochloride also known as vitamin $B_6$ hydrochloride or vitamin $B_6$ which is e.g. available as Pyridoxine hydrochloride or Pyridoxine Hydrochloride 98 DC at DSM Nutritional Products AG, (4303 Kaiseraugst, Switzerland).

The term 'an effective amount' as used herein refers to an amount necessary to obtain the physiological effect. The physiological effect may be achieved by one application dose or by repeated applications. The dosage administered may, of course, vary depending upon known factors, such as the physiological characteristics of the cosmetic composition comprising the respective extract and its mode and route of administration; the age, the nature and extent of the symptoms; the kind of concurrent treatment; the frequency of treatment; and the effect desired and can be adjusted by a person skilled in the art.

Preferably, the amount of the Vitamin $B_3$ such as in particular niacinamide in the compositions according to the present invention is selected in the range of 0.5 to 10 wt. %, preferably in the range of 1 to 10 wt. %, most preferably in the range of 1 to 5 wt. %, based on the total weight of the composition.

Preferably, the amount of Vitamin $B_6$ such as in particular pyridoxine hydrochloride in the compositions according to the present invention is selected in the range of 0.02 to 6 wt. %, preferably in the range of 0.05 to 4wt. %, most preferably in the range of 0.1 to 3 wt. %, based on the total weight of the composition.

In an advantageous embodiment, the amount of niacinamide is higher than the amount of pyridoxine hydrochloride. In a preferred embodiment, the ratio (w/w) of niacinamide to pyridoxine hydrochloride is selected in the range of 10 to 1 to 1.5 to 1, such as in the range of 5 to 1 to 2 to 1, such as 3 to 1.

The term 'cosmetic composition' as used herein refers to compositions, which are used to treat, care for or improve the appearance of the skin and/or the scalp. Particularly advantageous cosmetic compositions according to the present invention are skin care preparations.

The term 'cosmetically acceptable carrier' (also referred to herein as carrier) refers to all vehicles/ carriers conventionally used in cosmetic compositions, i.e. which are suitable for topical application to the keratinous tissue, have good aesthetic properties, are compatible with the actives present in the composition, and will not cause any unreasonable safety or toxicity concerns. Such carriers are well-known to one of ordinary skill in the art.

The exact amount of carrier will depend upon the actual level of the active ingredients and of any other optional ingredients that one of ordinary skill in the art would classify as distinct from the carrier (e.g., other active ingredients).

In an advantageous embodiment, the cosmetic compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In an advantageous embodiment, the carrier consists furthermore of at least 40 wt.-%, more preferably of at least 50 wt.-%, most preferably of at least 55 wt.-% of water, such as in particular of about 55 to about 90 wt.-% of water.

The compositions used in the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions may also comprise further cosmetically active ingredients conventionally used in cosmetic compositions. Exemplary active ingredients encompass skin lightening agents; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic compositions used in the present invention can be in a wide variety of forms. Non limiting examples include simple solutions (e.g. aqueous, organic solvent, or oil based), emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. of oil-in-water-in oil (O/W/O) or water-in-oil-in-water (W/O/W) type) or pickering emulsions), as well as solid forms (e.g. hydrogels, alcoholic gels, lipogels, sticks, flowable solids, or amorphous materials).

These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art.

If the composition is an emulsion, such as in particular an O/W-, W/O-, Si/W-, W/Si-, O/W/O-, W/O/W- or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the composition.

In one embodiment, the compositions used in the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the composition used in the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol® A from DSM Nutritional Products Ltd.), diethanolamine cetyl phosphate (e.g. as Amphisol® DEA from DSM Nutritional Products Ltd.), potassium cetyl phosphate (e.g. as Amphisol® K from DSM Nutritional Products Ltd.), sodium cetearylsulfate, sodium glyceryl oleate phosphate, hydrogenated vegetable glycerides phosphate and mixtures thereof. Further suitable emulsifiers are polyalkylene glycol ethers, sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the composition.

Particular suitable O/W emulsifiers to be used in the compositions used in the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate as well as polyalkylene glycol ethers such as in particular polyethylene stearyl ethers such as Steareth-2 and Steareth 21.

A particular suitable class of O/W emulsifier to be used in the compositions used in the invention are the cetyl phosphates such as in a particular potassium cetyl phosphate available at DSM Nutritional Products under the tradename Amphisol K.

In one particular embodiment, the invention relates to compositions with all the definitions and preferences given herein in the form of O/W emulsions comprising an oily phase dispersed in an aqueous phase in the presence of at least one O/W emulsifier wherein the at least one O/W emulsifier is potassium cetyl phosphate.

The cosmetic compositions used in the present invention advantageously comprise a preservative. Particular suitable preservatives in all embodiments of the present invention are phenoxyethanol and ethylhexylglycerin as well as mixtures thereof. When present, the preservative is preferably used in an amount of 0.1 to 2 wt.-%, more preferably in an amount of 0.5 to 1.5 wt.-%, based on the total weight of the composition.

The compositions used in the invention in general have a pH in the range of 3 to 10, preferably a pH in the range of 4 to 8 and most preferably a pH in the range of 5 to 8. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The amount of the compositions to be applied to the skin is not critical and can easily be adjusted by a person skilled in the art. Preferably the amount is selected in the range of 0.1 to 3 mg/ cm$^2$ skin, such as preferably in the range of 0.1 to 2 mg/ cm$^2$ skin and most preferably in the range of 0.5 to 2 mg / cm$^2$ skin.

The following examples are provided to further illustrate the compositions and effects of the present invention.

**Example**

Following the paper of T. Rudolph et. al. (SOFW-Journal, 140, 3-2014), beta-carotene (DSM, β-Carotene Crystalline, Lot Nr. WC01503161) was dissolved in o-xylene to a concentration of 0.5% (w/w) and was homogenously applied over a PMMA plate (Schönberg, sandblasted, $2\mu$m roughness) with a syringe. After 10min drying (RT, in the dark) the formulation as outlined in table 2 were applied ($2\mu$Lcm$^{-2}$) and distributed with a finger. Per formulation 3 replicates were produced and irradiated. Parallel 3 replicates with placebo cream (no active) were prepared which were also irradiated. The irradiation took place in an Atlas SunTester XLS+ with a total irradiance of 500Wm$^{-2}$ with an irradiation dose of 3MED. On top of the PMMA plates a UV-cut-off filter has been placed. This UV-cut-off filter is a Schott GG400-3.

After irradiation, the respective PMMA plates were extracted with 50mL isopropanol in an ultrasonic bath for 1 min and the residual amount of beta-carotene was photometrically quantified at 452nm.

Then the protection (%) was determined using the following formula:

$$\% \text{ Protection} = ([(\text{Absorbance sample}) * 100]/ [\text{Absorbance placebo}]-100)$$

(The absorbance at 452nm after irradiation of the placebo (maximum degradation) was set to 0%).

**Table 1: Results**

| Sample (active) | Protection* |
|---|---|
| 1 (none, Placebo) | 0% |
| 2 (3% Niacinamide) | +12% |
| 3 (1% Pyridoxin HCl) | +10% |
| 4 (3% Niacinamid + 1% Pyridoxin HCl) | +58% |
| *Increase in % versus placebo (irradiated) | |

As can be retrieved from table 1, the addition of the combination of niacinamide and Pyridoxin hydrochloride to the placebo formulation led to a synergistic protection of the beta-carotene from blue light degradation.

**Table 2**

| Sample<br>INCI name | 1 | 2 | 3 | 4 |
|---|---|---|---|---|
| BIS-ETHYLHEXYLOXYPHENOL METHOXYPHENYL TRIAZINE | 1.5 | 1.5 | 1.5 | 1.5 |
| POLYSILICONE-15 | 1 | 1 | 1 | 1 |
| BUTYL METHOXYDIBENZOYLMETHANE | 2 | 2 | 2 | 2 |
| ETHYLHEXYL SALICYLATE | 5 | 5 | 5 | 5 |
| OCTOCRYLENE | 1.5 | 1.5 | 1.5 | 1.5 |
| DIISOPROPYL SEBACATE | 3 | 3 | 3 | 3 |
| DIMETHICONE | 2 | 2 | 2 | 2 |
| DICAPRYLYL ETHER | 2 | 2 | 2 | 2 |
| DICAPRYLYL ETHER | 2 | 2 | 2 | 2 |
| TITANIUM DIOXIDE, SILICA, DIMETHICONE | 3 | 3 | 3 | 3 |
| CETYL PHOSPHATE | 1.5 | 1.5 | 1.5 | 1.5 |
| STEARYL ALCOHOL | 3.15 | 3.15 | 3.15 | 3.15 |
| HYDROXYETHYL ACRYLATE/SODIUM ACRYLOYLDIMETHYL TAURATE COPOLYMER | 0.5 | 0.5 | 0.5 | 0.5 |
| POLYACRYLATE CROSSPOLYMER-6 | 0.5 | 0.5 | 0.5 | 0.5 |
| PHENOXYETHANOL, ETHYLHEXYLGLYCERIN | 1 | 1 | 1 | 1 |
| SILICA | 3 | 3 | 3 | 3 |
| AQUA | Ad 100 | | | |
| PROPANEDIOL | 5 | 5 | 5 | 5 |
| TROMETHAMINE, AQUA | 0.86 | 0.86 | 0.86 | 0.86 |
| METHYLENE BIS-BENZOTRIAZOLYL TETRAMETHYLBUTYLPHENOL, AQUA | 8 | 8 | 8 | 8 |
| NIACINAMIDE | | 3 | | 3 |
| PYRIDOXIN HCI | | | 1 | 1 |

**Claims**

1. Non-therapeutic use of a cosmetic composition containing an effective amount of Vitamin $B_3$ and an effective amount of Vitamin $B_6$ for protecting human skin against damage upon exposure to blue light.

2. The use according to claim 1, wherein the Vitamin $B_3$ is niacinamide and the Vitamin $B_6$ is pyridoxine hydrochloride.

3. The use according to any one of claims 1 to 2, wherein the effective amount of the Vitamin $B_3$ or derivative thereof is selected in the range of 0.5 to 10 wt. %, preferably in the range of 1 to 10 wt. %, most preferably in the range of 1 to 5 wt. %, based on the total weight of the composition.

4. The use according to any one of claims 1 to 3, wherein the effective amount of the Vitamin $B_6$ or derivative thereof is selected in the range of 0.02 to 6 wt. %, preferably in the range of 0.05 to 4 wt. %, most preferably in the range of 0.1 to 3 wt. %, based on the total weight of the composition.

5. The use according to any one of claims 1 to 4, wherein the amount of niacinamide is higher than the amount of pyridoxine hydrochloride.

6. The use according to any one of claim 1 to 5, wherein the damage is the result of the generation of reactive oxygen species, reactive nitrogen species and/ or reactive carbonyl species.

7. The use according to any one of claims 1 to 6, wherein the composition is a skin care composition.

8.  The use according to anyone of claims 1 to 7, wherein the composition is in the form of O/W emulsions comprising an oily phase dispersed in an aqueous phase in the presence of an O/W emulsifier.

9.  The use according to claim 8, **characterized in that** the O/W emulsifier is potassium cetyl phosphate.

10. Non.therapeutic use of a cosmetic composition containing an effective amount of niacinamide and pyridoxine hydrochloride for reducing the generation of reactive oxygen species and/or the formation of carbonylated proteins in humans when expposed to blue light.

11. The use according to any one of claims 1 to 10, wherein the effective amount of the niacinamide in the cosmetic composition is selected in the range of 0.5 to 10 wt. %, and the amount of pyridoxine hydrochloride is selected in the range of 0.02 to 6 wt. %, based on the total weight of the cosmetic composition.

**Patentansprüche**

1.  Nichttherapeutische Verwendung einer kosmetischen Zusammensetzung, die eine wirksame Menge von Vitamin $B_3$ und eine wirksame Menge von Vitamin $B_6$ enthält, zum Schutz von menschlicher Haut von Schädigung bei Exposition gegenüber blauem Licht.

2.  Verwendung gemäß Anspruch 1, wobei das Vitamin $B_3$ Niacinamid ist und das Vitamin $B_6$ Pyridoxinhydrochlorid ist.

3.  Verwendung gemäß einem der Ansprüche 1 bis 2, wobei die wirksame Menge des Vitamins $B_3$ oder Derivats davon ausgewählt ist aus dem Bereich von 0,5 bis 10 Gew.-%, vorzugsweise aus dem Bereich von 1 bis 10 Gew.-%, höchst bevorzugt aus dem Bereich von 1 bis 5 Gew.-%, bezogen auf das Gesamtgewicht der Zusammensetzung.

4.  Verwendung gemäß einem der Ansprüche 1 bis 3, wobei die wirksame Menge des Vitamins $B_6$ oder Derivats davon ausgewählt ist aus dem Bereich von 0,02 bis 6 Gew.-%, vorzugsweise aus dem Bereich von 0,05 bis 4 Gew.-%, höchst bevorzugt aus dem Bereich von 0,1 bis 3 Gew.-%, bezogen auf das Gesamtgewicht der Zusammensetzung.

5.  Verwendung gemäß einem der Ansprüche 1 bis 4, wobei die Menge an Niacinamid größer als die Menge an Pyridoxinhydrochlorid ist.

6.  Verwendung gemäß einem der Ansprüche 1 bis 5, wobei die Schädigung die Folge der Erzeugung von reaktiven Sauerstoffspezies, reaktiven Stickstpoffspezies und/oder reaktiven Carbonylspezies ist.

7.  Verwendung gemäß einem der Ansprüche 1 bis 6, wobei die Zusammensetzung eine Hautpflegezusammensetzung ist.

8.  Verwendung gemäß einem der Ansprüche 1 bis 7, wobei die Zusammensetzung in der Form von O/W-Emulsionen vorliegt, die eine ölige Phase in Gegenwart eines O/W-Emulgators in einer wässrigen Phase dispergiert umfasst.

9.  Verwendung gemäß Anspruch 8, **dadurch gekennzeichnet, dass** der O/W-Emulgator Kaliumcetylphosphat ist.

10. Nichttherapeutische Verwendung einer kosmetischen Zusammensetzung, die eine wirksame Menge von Niacinamid und Pyridoxinhydrochlorid enthält, zum Verringern der Erzeugung von reaktiven Sauerstoffspezies und/oder der Entstehung vom carbonylierten Proteinen in Menschen bei Exposition gegenüber blauem Licht.

11. Verwendung gemäß einem der Ansprüche 1 bis 10, wobei die wirksame Menge des Niacinamids in der kosmetischen Zusammensetzung ausgewählt ist aus dem Bereich von 0,5 bis 10 Gew.-% und die Menge an Pyridoxinhydrochlorid ausgewählt ist aus dem Bereich von 0,02 bis 6 Gew.-%, bezogen auf das Gesamtgewicht der kosmetischen Zusammensetzung.

**Revendications**

1.  Utilisation non thérapeutique d'une composition cosmétique contenant une quantité efficace de vitamine $B_3$ et une quantité efficace de vitamine $B_6$ pour protéger la peau humaine contre une lésion lors d'une exposition à de la lumière bleue.

2. Utilisation selon la revendication 1, la vitamine $B_3$ étant le niacinamide et la vitamine $B_6$ étant le chlorhydrate de pyridoxine.

3. Utilisation selon l'une quelconque des revendications 1 à 2, la quantité efficace de la vitamine $B_3$ ou d'un dérivé correspondant étant choisie dans la plage de 0,5 à 10 % en poids, préférablement dans la plage de 1 à 10 % en poids, le plus préférablement dans la plage de 1 à 5 % en poids, sur la base du poids total de la composition.

4. Utilisation selon l'une quelconque des revendications 1 à 3, la quantité efficace de la vitamine $B_6$ ou d'un dérivé correspondant étant choisie dans la plage de 0,02 à 6 % en poids, préférablement dans la plage de 0,05 à 4 % en poids, le plus préférablement dans la plage de 0,1 à 3 % en poids, sur la base du poids total de la composition.

5. Utilisation selon l'une quelconque des revendications 1 à 4, la quantité de niacinamide étant supérieure à la quantité de chlorhydrate de pyridoxine.

6. Utilisation selon l'une quelconque des revendications 1 à 5, la lésion étant le résultat de la génération d'espèces d'oxygène réactives, d'espèces d'azote réactives et/ou d'espèces de carbonyle réactives.

7. Utilisation selon l'une quelconque des revendications 1 à 6, la composition étant une composition de soins de la peau.

8. Utilisation selon l'une quelconque des revendications 1 à 7, la composition étant sous la forme d'émulsions H/E comprenant une phase huileuse dispersée dans une phase aqueuse en la présence d'un émulsifiant H/E.

9. Utilisation selon la revendication 8, **caractérisée en ce que** l'émulsifiant H/E est le cétylphosphate de potassium.

10. Utilisation non thérapeutique d'une composition cosmétique contenant une quantité efficace de niacinamide et de chlorhydrate de pyridoxine pour la réduction de la génération d'espèces d'oxygène réactives et/ou la formation de protéines carbonylées chez des humains lorsqu'ils sont exposés à de la lumière bleue.

11. Utilisation selon l'une quelconque des revendications 1 à 10, la quantité efficace du niacinamide dans la composition cosmétique étant choisie dans la plage de 0,5 à 10 % en poids, et la quantité de chlorhydrate de pyridoxine étant choisie dans la plage de 0,02 à 6 % en poids, sur la base du poids total de la composition cosmétique.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9730690 A
- US 2005100517 A

**Non-patent literature cited in the description**

- *Journal of the American Academy of Dermatology,* 2010, vol. 63 (3), 507-525
- **HAKOZAKI et al.** *British Journal of Dermatology,* 2002, vol. 147 (1), 20-31
- *CHEMICAL ABSTRACTS,* 66-72-8
- *CHEMICAL ABSTRACTS,* 85-87-0
- **T. RUDOLPH.** *SOFW-Journal,* March 2014, vol. 140